# EUROPEAN PATENT APPLICATION

(11) **EP 4 449 877 A1**
(43) Date of publication of application: **23.10.2024**
(21) Application number: 24166981.1
(22) Date of filing: 27.03.2024
(51) Int. Cl.: A23K 10/20, A01K 67/033, A23K 50/40, A23P 30/00

(54) **LIQUEFIED FEED MATERIAL FROM HERMETIA ILLUCENS**

(30) Priority: 17.04.2023 PL 44443823
(71) Applicant: HiProMine S.A., 62-023 Robakowo (PL)
(72) Inventor: Dudek, Krzysztof, 64-552 Sedziny (PL); Sypniewski, Jedrzej, 62-040 Puszczykowo (PL); Staniszewski, Marcin, 61-491 Poznan (PL); Janicki, Bartosz, 61-249 Poznan (PL); Ciarka, Kamil, 61-619 Poznan (PL); Józefiak, Damian, 60-479 Poznan (PL)
(74) Representative: Grzelak, Anna

(57) **Abstract**

The invention relates to a method for producing a liquefied feed material obtained from the larvae of insects belonging to *Hermetia illucens* species on a technological line, to the liquefied feed material obtained from insect larvae and produced by this method, and to its use in production of food for companion animals, especially for dogs and cats.

## Description

### TECHNICAL FIELD

The invention relates to a method for producing a liquefied feed material obtained from the larvae of insects belonging to *Hermetia illucens* species on a technological line, to the feed material produced by this method, and to its use in the production of food for companion animals, especially for dogs and cats.

### STATE OF THE ART

Use of feed materials from insects has scientifically proven advantages from both nutritional and environmental points of view. Research has shown that their use in the feeding of livestock and pets translates into better animal health and growth parameters thanks to the presence of anti-inflammatory and anti-bacterial substances. Due to their hypoallergenicity, they are used in food for dogs and other companion animals, where they successfully replace vertebrate meat. The breeding and further processing of insects is environmentally sustainable and meets the principles of a circular economy, which is very important for ecological reasons and the proper use of resources. Insect production does not use potable water, is not energy-intensive and does not require as much space as the production of standard feed materials. Moreover, the production is waste-free and, by using by-products from agri-food production, it contributes to reducing biological waste on the market by converting it into valuable products.

Insect products are replacing standard materials, i.e., soybean meal and fish meal in livestock feed and vertebrate meat in companion animal food. Insect products are characterised by a high protein content in the dry matter with simultaneously reduced fat levels and maintained homogeneity, which results in the advantage of a higher nutrient concentration, contributing to lower feed application costs. Insects also have more exogenous amino acids, a high level of fat-soluble vitamins of the ADEK group, which reduces the costs of supplementation for animals. It is important that insect products contain anti-inflammatory and immunomodulatory substances and antimicrobial proteins (AMPs), which results in improved economic indicators in animal rearing and reduced chemoprophylaxis, lower morbidity, better health, improved feed conversion rates and weight gains. Immunostimulatory effect reduces biochemical inflammatory markers in blood while improving the body's immune response. In the case of food for companion animals, especially for dogs and cats, the hypoallergenicity of insect protein is important, so insect feed can be used in specialized, veterinary and elimination diets.

The most innovative product is insect meat that is the equivalent of vertebrate meat. It is used in animal food where it is a substitute for premium products such as lamb, rabbit meat, game meat or beef. Thanks to the positive environmental aspects of insect breeding, its use results in a significant reduction in carbon and water footprint of the final product. In addition to the above-mentioned nutritional advantages, insect meat, which is a minimally processed material that retains the smell and taste of insects, has a high palatability, as shown by studies on dogs and cats where insect meat-based food were willingly chosen. As a result, insects can be used not only in complete food but also in animal treats.

The industrial production of fresh insect meat poses technological problems that have so far limited the supply of this type of material. The very first is the problem of rapid oxidation of the material, which leads to a change in colour to dark brown to black and a change in smell to an unpleasant one. Oxidised, dark-coloured product is reluctantly purchased by consumers because it influences the quality of the final product: animal food, especially for dogs or cats. Rapid oxidation also reduces the vitamin content in insect meat, including ADEK group vitamins, which are particularly valuable. The second problem in fresh meat production is its rapid contamination with pathogenic microorganisms such as *Salmonella spp.* genus and Enterobacteriaceae (Enterobacteriaceae family). This necessitates the addition of preservatives, which, however, has a negative impact on the perception of the product, which by definition is presented as natural and free from chemical additives.

Installations used for the production of food for companion animals, especially dry food for dogs or cats, require the supply of an intermediate product in a liquefied form having a specified viscosity together with an adequate quantity and quality of a liquefied meat product, having a viscosity of 30-50 Pa*S measured at 30°C. Therefore, the methods known to date for obtaining liquefied feed material from insects from fresh meat of *Hermetia illucens* did not meet all the requirements set by the recipients of such installations, in particular they did not simultaneously ensure high quality of the liquefied product, its microbiological purity, and suitable density as well as other parameters that would allow it to be used on the same lines that are used for the production of food for companion animals, especially for dogs and cats, especially in dry form.

### DISCLOSURE OF THE INVENTION

The aim of the invention is to overcome the indicated disadvantages known from the prior art. This aim has been achieved by developing a method for producing improved liquefied feed material from insects on a technological module developed for this purpose.

The invention relates to a method for producing liquefied feed material from *Hermetia illucens* insect biomass comprising the following steps in which the input raw material in the form of 10-14-day-old *Hermetia illucens* larvae, preferably 10-day-old *Hermetia illucens* larvae, is fed onto a drum screen (trommel screen), on which in step A, waste material smaller than 3.5 × 3.5 mm is separated by sieving and shaking, and they are then transferred, preferably by a belt conveyor, to a belt-type immersion washer, in which in step B, the larvae are washed and soaked in water at a temperature of 5-20°C, preferably at a temperature of 6-10°C, for a period of 20 seconds to 10 minutes, preferably for 30 seconds to 2 minutes, and then they are transported by a conveyor, preferably a belt conveyor, to a blanching machine 3, where in step C, the larvae are blanched and sterilised by rapid immersion and heating in water heated to 75-95°C, preferably 80-90°C, and kept at this temperature for 6-10 minutes, preferably 7-9 minutes, preferably 8 minutes, and then they are transferred by a conveyor, preferably a belt conveyor, to a cooler 4 in which in step D, the larvae are rapidly cooled to a temperature of 4-6°C, preferably to 5°C, and then they are transferred by a conveyor, preferably a vibratory conveyor, preferably a belt conveyor, to a flow-through cutter, wherein the mass of larvae fed into the flow-through cutter is simultaneously liquefied by the addition of ice water and/or ice from a mixer to achieve a dry matter of 15-30%, and in the subsequent step E, a flow-through grinding and homogenisation process of larvae is performed to obtain liquefied feed material from insect biomass with solid particle diameter granulation fraction of up to 1 mm.

In a preferred method, the liquefied feed material from insect biomass prepared in step E is then transferred, preferably by a pipeline, to a receiving and packaging station; at the receiving and packaging station, the liquefied feed material from insect biomass is preferably packaged into containers, preferably bulk containers, or from the receiving and packaging station, the liquefied feed material from insect biomass is preferably transferred, preferably by a pipeline, to a production belt of a technological line used for the production of food for companion animals, where food for companion animals, preferably dogs and cats, is produced.

In a preferred method, water circulation system of the belt-type immersion washer is a closed system in which dirty water is filtered and reused for washing in the belt-type immersion washer in the washing and soaking step B.

In a preferred method, the water circulation system in the blanching machine is a closed system in which water is filtered, reheated and reused in the blanching step C.

In a preferred method, microbiologically pure, preferably sterile, water is used to produce water and/or ice in the mixer.

In a preferred method in step A, the waste material is removed by a conveyor belt.

In a preferred method, the waste material, which is contaminants, manure and too small larvae, is then processed to produce a fertiliser, preferably as frass, which is preferably transferred to a fertiliser production technological line and the waste material is processed to produce a fertiliser, preferably as frass.

The invention also relates to liquefied feed material from *Hermetia illucens* insect biomass produced by the method according to the invention.

The liquefied feed material from *Hermetia illucens* insect biomass has the following parameters: dry matter is 15-30% w, it has a density in the range of 0.836-0.940 g/ml, has a viscosity at a temp. of 30°C in the range of 30-50 Pa*S, has a solid particle size diameter of up to 1 mm, total bacterial count is up to 10⁵ CFU/g.

The invention also relates to the use of the liquefied feed material from *Hermetia illucens* insect biomass for the production of food for companion animals, preferably dogs and cats.

In a preferred use, the liquefied feed material from *Hermetia illucens* insect biomass is used in an amount of 10-50% w/w based on the total weight of the food.

In a preferred use, the food is in a semi-liquid, solid, preferably dry form, preferably in the form of an animal treat.

Unexpectedly, the inventors of the present solution found that by using specific successive steps with strictly selected parameters on the developed technological module, it is possible to obtain an improved liquefied insect material that is essentially non-oxidised and has suitable taste and physico-chemical parameters (moisture content, density, viscosity, colour, smell) enabling it to be use in the already existing installations used for the production of companion animal food, especially in dry and semi-dry form.

The methods known to date for obtaining liquefied feed material from insects from fresh meat of *Hermetia illucens* did not provide suitable qualitative (absence of oxidation, light colour, high level of ADEK group vitamins), physico-chemical properties (viscosity, qualitative and quantitative composition, quantity of meat g/dm³, meat particle size in the liquefied material, its uniformity (homogeneity) and no separation into layers of the resulting suspension of the liquefied insect pulp, which behaves like a colloidal solution long enough to carry out the processing during the production of the food for accompanying animals).

Among others, the applied an immersion pre-washing step ensuring simultaneous pre-soaking of larvae with subsequent rapid thermal treatment with appropriate liquefaction of the larvae, together with rapid cooling of the larvae prior to the homogenisation step in order to obtain a liquefied insect pulp as a substitute for mechanically separated meat (MSM) to obtain a high-quality liquefied intermediate product suitable for use in the already existing production lines producing the food for companion animals, in particular dogs and cats, was found to be preferable. Before being introduced onto the production line used for the production of companion animal food, such a feed material did not require any additional treatment, such as additional liquefaction, homogenisation, and it had a suitable viscosity, and although it contained a lot of meat material per volume unit, it did not cause clogging of the ducts transporting the liquefied feed material from insect biomass according to the invention onto the production line used for the production of companion animal food.

Innovative research carried out by the inventors has shown that it is possible to obtain a ground and liquefied as well as non-oxidised insect mass that retains a light colour indicating the absence of oxidation and will be preserved by sterilisation without the use of preservative additives. Among others, the use of a two-step cleaning of the larvae: firstly, dry cleaning on drum screens and then wet cleaning in a water washer, preceding the thermal treatment process, proved to be crucial in obtaining feed material from insect biomass with such properties. Pre-soaked during wet cleaning, the larvae are blanched by exposure to water at a temperature in the range of 75-95°C for preferably 6-10 minutes, preferably about 8 minutes so that all pathogenic microorganisms are neutralised. Surprisingly, the blanching process, which is conducted after pre-soaking the larvae in the immersion washing step, can be short and leads to the preservation of insect proteins and other substances such as vitamins, fats, so that the insect pulp does not oxidise after grinding and retains a light, creamy colour and at the same time the material is not subject to 'overcooking'. It is crucial for the production of a material that does not undergo oxidation to combine all processes, including the use of a rapid cooling in a cooler and of a mixer that allows cold or ice water to be added before the homogenisation step to supplement the larvae mass with water, thus obtaining a pulp that, after the step of homogenisation to a fraction with a maximum particle diameter of 1 mm, is adequately liquefied and can therefore be used in companion animal food production lines without further treatment. Before the addition of ice/ice water, the pulp contains about 35-50% dry matter and the final product obtained after homogenisation has 15-25% dry matter and is suitable for direct use in technological lines used for the production of food for companion animals.

In summary, the essence I of the invention is to perform specific operations in a particular order for the purpose of treating insect material on a suitably adapted technological module that ensures cleaning of the larvae, thermal treatment and homogenisation together with the liquefaction of *Hermetia illucens* fly larvae delivered from the production line, which leads to obtaining a liquefied feed material from insect biomass that has improved physico-chemical properties, is non-oxidised, has an attractive light colour, contains non-oxidised proteins, fats and vitamins, is microbiologically pure, does not need to be supplemented with antioxidant or preservative chemical additives or with antibacterial and fungicidal agents, has a suitable viscosity in the range of 30-50 Pa*S and a density in the range of 0.836-0.940 g/ml, making it possible to use the feed material from insect biomass in the already existing installations used for the production of companion animal food, especially in dry and semi-dry form. The resulting product in the form of liquefied feed material from *Hermetia illucens* insect biomass does not require freezing and can be stored at 4°C due to the microbiological purity achieved.

### BRIEF DESCRIPTION OF THE DRAWING

Non-limiting embodiments of the invention are shown in Figures, in which:

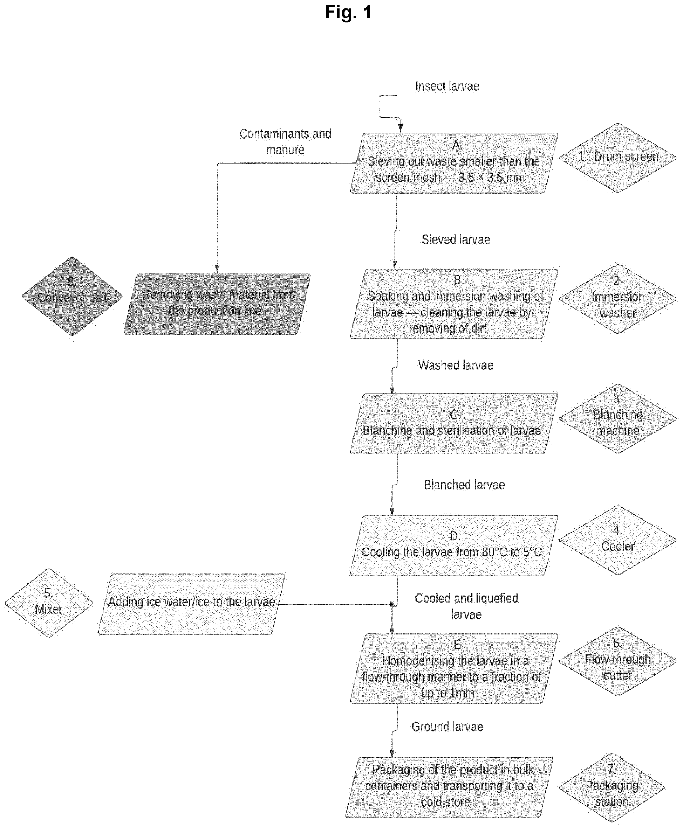

**Fig. 1****.** provides a schematic representation of the individual steps and the equipment used therein in the method of producing liquefied feed material from *Hermetia illucens* insect biomass (insect pulp) on a production line used for the production of liquefied insect pulp.

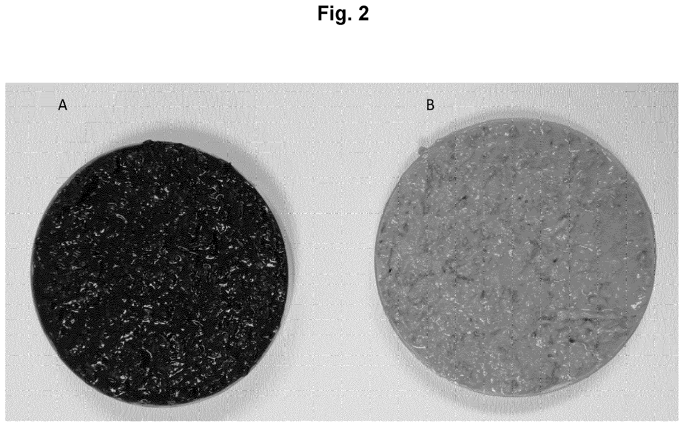

**Fig. 2****.** shows a (magnified) photograph of a portion of an insect pulp prepared by a standard method and having a dark brown, almost black colour resulting from strong oxidation of the material (A), and a photograph of the liquefied feed material from *Hermetia illucens* insect biomass prepared by the method according to the invention and having a light beige colour, in the form of an essentially non-oxidised material (B).

### DESCRIPTION OF EMBODIMENTS

The following examples are included only to illustrate the invention and to explain its particular aspects, not to limit the invention, and should not be construed as the entire scope thereof that is defined in the appended claims.

### EXAMPLES

### Example 1. Technological module for the production of an improved liquefied insect pulp

A prototype production line was created as a technological module added to a known and previously disclosed production line. The prepared prototype was firstly created at laboratory scale, testing different sequences of operations and their conditions, and then scaled up to a production scale to confirm the feasibility of achieving the intended objective and to automate and ensure the continuity of the production process of liquefied insect pulp at production scale.

The pilot semi-production module confirmed the feasibility of scaling up the proposed method of processing of insect material from *Hermetia illucens* and of providing liquefied insect pulp (liquefied feed material from insect biomass) with suitable moisture content parameters, very reduced oxidation of the final product, suitable organoleptic parameters, and suitable viscosity, even when it was produced at semi-production scale, while maintaining the intended target parameters for continuous processing.

The pilot technological module at semi-production scale was a system of equipment designed to process raw material in the form of *Hermetia illucens* insect larvae to obtain final products with the desired qualitative and quantitative parameters.

The module includes the following devices and their assemblies (see **Fig. 1**), from the entry of the material from the production line: the larvae are transferred, preferably by a belt conveyor, to a drum screen 1 - which ensures the separation of waste smaller than the screen mesh of e.g. 3.5 × 3.5 mm, and from which the sieved larvae are transferred, preferably by a belt conveyor, to a belt-type immersion washer 2 - for cleaning the larvae from dirt and for soaking them by immersion soaking, from which the washed and soaked larvae are transferred, preferably by a belt conveyor, to a blanching machine 3 - where the larvae are rapidly heated/blanched and sterilised; from the blancher the sterilised and blanched larvae are transferred, preferably by a belt conveyor, to a cooler 4, from which the cooled larvae are transferred, preferably by a belt conveyor, to a rapid-cooling installation with mixing with homogenisation/grinding, which installation is a grinder 6 and a mixer 5, the larvae being delivered to the grinder 6, i.e. in this embodiment: a pass-through cutter 6, that provides a flow-through grinding and homogenisation process for grinding the larvae in a flow-through manner with simultaneous supplementation with ice water and/or ice from the ice/ice water mixer 5 during the feeding of the larvae to the pass-through cutter 6, where the larvae are rapidly ground (comminuted) in a flow-through manner to a fraction with a maximum of 1 mm solid particle diameter, from which the liquefied feed material from insect biomass is fed, preferably by a pipeline, to a receiving and packaging station 7 to be packed into transport containers or directly to production belt on technological lines used for the production of companion animal food.

In addition, microbiologically pure, preferably previously boiled or filtered water is used in the technological module in the ice/ice water mixer 5 to ensure microbiological purity of the final product.

The input raw material was produced on a production line as described in patent PL230275. The input raw material was 10-14-day-old larvae, preferably 10-day-old *Hermetia illucens* larvae.

In step A, the larvae are separated from the production waste: contaminants and manure, which is removed by a discharge conveyor belt 8 and is removed as a waste material that can be used, e.g., to produce a fertiliser. Larvae that were too small, at the wrong growth stage or dead were sieved out on the drum screen 1 by sieving and shaking. *Hermetia illucens* larvae pre-cleaned on the screen 1 were fed by a conveyor to the washing machine - immersion belt washer 2 via an inclined conveyor. In the immersion washer 2 in step B, residual waste that could not be removed by sieving was removed from the larvae, while the larvae were pre-wetted/soaked. Water circulation system of the belt-type immersion washer 2 is a closed system - dirty water is filtered and reused for washing. Washed and soaked larvae are transferred by a belt conveyor to the blanching machine 3, where step C larvae sterilisation/blanching is carried out in heated water at a temperature in the range of 80-90°C for about 7-9 minutes, preferably for 8 minutes. Additionally, at the blanching step C, the constantly hot water is used in an essentially continuous and closed cycle, is subjected to a filtration process, so that it is not necessary to continuously replenish and heat water, but only to slightly warm it up to reach the technological parameters (the appropriate temperature). The sterilised/blanched hot larvae are transferred, preferably by a belt conveyor, to the cooler 4, where in step D, the temperature of the larvae drops rapidly to about 5°C. The cooler 4 was equipped with a plate heat exchanger - and the process water circulates in a closed system and is only cooled a little. Rapid transition from the blanching step C with a temperature of about 80-90°C to a temperature of about 5°C in the rapid cooling step D additionally influences the preparation of the essentially non-oxidised final product (**Fig. 2**).

Downstream of the cooler 4, an ice mixer 5 was placed, which allowed ice or ice water to be added to the raw material in order to achieve a suitable moisture content in the mass of the final product. Preferably, the additional cooling of larvae by the addition of ice or ice water improved the properties of the final liquefied insect pulp.

After cooling the larvae in step D and supplementing of the ice water/ice, they are passed onto a conveyor assembly to the vibratory conveyor, which dispenses larvae to the grinder, in this embodiment: the flow-through cutter 6, designed to process the larvae suspended in ice water into a liquefied insect pulp, which occurs in stage E of the process. Grinding (milling) of the larvae is carried out in a flow-through manner to obtain a fraction with a maximum diameter of 1 mm.

The final product in the form of liquefied insect pulp (liquefied feed material) is packaged at packaging station 7 into containers and sent to a cold store.

By conducting the process in a continuous way, the efficiency of the entire process at semi-industrial scale is about 2 tonnes per hour of raw material processed.

Thanks to the filtration system and the essentially closed water circuit at various steps of the process, the water consumption in the technological module have been significantly reduced, as well as the energy consumed in the process and needed to adapt its parameters to the technology.

The technological line thus constructed in this way resulted in a product: a liquefied insect pulp with a high fluidity, suitable viscosity, suitable microbiological purity and maintained nutritional composition. The product was characterised by the following features:
- Dry weight 20%;
- Maximum solid particle size 1 mm;
- Dry matter protein content 49.2 wt% (i.e. 49.2 g of protein in 100 g of dry matter);
- Dry matter fat content 25.6 wt%. (i.e. 25.6 g of fat in 100 g of dry matter);
- Ash in the dry matter 8.9 wt% (i.e. 8.9 g of ash per 100 g of dry matter);
- Fibre in the dry matter 13.8 wt% (i.e. 83.8 g of fibre in 100 g of dry matter)
- Viscosity 30-50 Pa*S at 30°C;
- Density 0.912 g/ml;
- Lauric acid content in fatty acid profile 40.3 wt% ;
- Monoproteinity

In contrast to standard insect pulp, which undergoes rapid oxidation the resulting material was light in colour (**Fig. 2**). The product was microbiologically pure according to the applicable standards (**Table 1**). During the analyses, the following were detected::

**Table 1. Analysis of microbiological purity of the final liquefied feed material from insect biomass**

| **Species [CFU/g] [colony forming units/g]** | **Value** |
|---|---|
| Clostridium perfringens | absent in 1 g |
| Enterobacteriaceae | <10³ CFU/g |
| Salmonella spp. | absent in 25 g |
| Lactic acid bacteria (LAB) | absent in 25 g |
| Total bacterial count | <10⁵ CFU/g |

The prepared technological module and method of producing liquefied insect pulp according to the invention contributed to improving the microbiological quality of the final product, i.e. it made it possible to develop best manufacturing practices in the inactivation of insect life processes and of microbial contaminants and to extend the shelf life of the produced insect biomass in the form of liquefied feed material without the use of preservatives. The liquefied insect pulp (liquefied feed material) produced in this technological module achieved the appropriate physico-chemical parameters (viscosity, chemical composition, particle size, moisture content, uniformity) for the production of companion animal food in the already existing adapted installations. The resulting liquefied insect feed material had improved microbiological parameters, was essentially not oxidised, the viscosity obtained allowed it to be used in the already existing installations used for the production of animal food, especially for dogs and cats.

A comparison of the characteristics was made between the liquefied insect feed material produced by the method of the invention (liquefied pulp) and produced by known techniques (standard pulp) (**Table 2**).

**Table 2. Comparison of the liquefied insect pulp (liquefied feed material) of the invention and a standard product: insect pulp produced by a standard method.**

| **FEATURE** | **LIQUEFIED PULP** (according to the invention) | **STANDARD PULP** (standard material) |
|---|---|---|
| Dry matter | 15-30% | 30-40% |
| Density | 0.836-0.940 q/ml | 1.000-1.343 |
| Viscosity at temp. of 30°C | 30-50 Pa*S | 60-80 Pa*S |
| Particle size | up to 1 mm (colloidal system) | 2-5 mm (mixture) |
| Colour (dependent on oxidation) | Light | Dark |
| Preservatives | None | Present |
| Storage temperature | 4°C | -20°C |
| Sterilisation | Heat | None |
| Are preservatives necessary to maintain microbiological purity | **No** preservatives added (the treatment used ensures sufficient microbiological purity and stability) | **Yes** - preservatives and optionally antibacterial/fungicidal agents are added |

### Example 2. Production of dog feed based on liquefied insect feed material

A dog food was produced in which the main source of protein was the liquefied insect pulp produced according to **Example 1,** a monoprotein feed material made from the larvae of insects of *Hermetia illucens* species. The food produced consisted of 50% of liquefied pulp, additionally contained 15% of vegetables, 5% of eggs, 3% of starch, 5% of animal and vegetable oils. Tests were carried out on industrial dog food production lines, where the standard material, i.e. mechanically separated meat (MSM), was replaced with liquefied insect pulp produced according to **Example 1.** The pumping of the product, i.e. liquefied pulp, has been tested in closed systems - pipelines with a diameter of 10 to 100 mm. Standard gear pumps and screw pumps were used for pressure generation and the product was transported over distances of up to 10 m. Dry matter of 20-24% led to adequate hydration of the product, resulting in a viscosity of 8-10 P*S and a density of 0.836-0.940 g/ml. This enabled both trouble-free pumping in the installation using standard pumps and no clogging of the extruders in the food production line.

The performance tests carried out have confirmed the feasibility of using such liquefied pulp in the form of liquefied feed material from *Hermetia illucens* insect biomass according to the invention as a substitute for MSM without limiting the efficiency of the production process for the production of animal food, especially for dogs and cats.

A test quantity of dry dog food was produced.

The dry dog food produced was characterised by high quality parameters and was readily eaten by dogs.

### LIST OF REFERENCES:

- 1: Drum screen (trommel screen)
- 2: Belt-type immersion washer
- 3: Blanching machine
- 4: Cooler - rapid-cooling system
- 5: Water and ice mixer
- 6: Grinder - larvae grinding flow-through cutter
- 7: Receiving and packing station
- 8: Conveyor belt for discharging production waste

## Claims

1. A method for producing liquefied feed material from *Hermetia illucens* insect biomass **characterised in that** it comprises the following steps in which
the input raw material in the form of 10-14-day-old *Hermetia illucens* larvae, preferably 10-day-old *Hermetia illucens* larvae, is fed onto a drum screen (1), on which in step (A), waste material smaller than 3.5 × 3.5 mm is separated by sieving and shaking, and the larvae are then transferred, preferably by a belt conveyor, to
a belt-type immersion washer (2), in which in step (B), the larvae are washed and soaked in water at a temperature of 5-20°C for a period of 0.5 to 10 minutes, and then the larvae are transported by a conveyor, preferably by a belt conveyor, to
a blanching machine (3), where in step (C), the larvae are blanched and sterilised by rapid heating in water at a temperature of 75-95°C, preferably 80-90°C, and kept at this temperature for 6-10 minutes, preferably 7-9 minutes, preferably 8 minutes, and then they are transferred by a conveyor, preferably by a belt conveyor, to
a cooler (4) in which in step (D), the larvae are rapidly cooled to a temperature of 4-6°C, preferably to 5°C, and then the larvae are transferred by a conveyor, preferably a vibratory conveyor, preferably by a belt conveyor, to
a flow-through cutter (6), wherein the mass of larvae fed into the flow-through cutter (6) is simultaneously liquefied by the addition of ice water and/or ice from an ice water and/or ice mixer (5) to achieve a dry matter of 15-30% w/w, and in step (E), a flow-through grinding and homogenisation of larvae is performed to obtain liquefied feed material from insect biomass with solid particle diameter granulation fraction of up to 1 mm.

2. The method according to claim 1, **characterised in that** the liquefied feed material from insect biomass obtained in step (E) is subsequently transferred, preferably by a pipeline, to a receiving and packaging station (7).

3. The method according to claim 2, **characterised in that** at the receiving and packaging station (7), the liquefied feed material from insect biomass is packaged into containers, preferably bulk containers.

4. The method according to claim 2, **characterised in that** from the receiving and packaging station (7), the liquefied feed material from insect biomass is transferred, preferably by a pipeline, to a production belt of a technological line used for the production of food for companion animals, where food for companion animals, preferably dogs and cats, is produced.

5. The method according to claim 1-4, **characterised in that** water circulation system of the belt-type immersion washer (2) is a closed system in which dirty water is filtered and reused for washing in the belt-type immersion washer (2) in the larvae washing and soaking step (B).

6. The method according to claim 1-5, **characterised in that** the water circulation system in the blanching machine (3) is a closed system in which water is filtered, reheated and reused in the blanching step (C).

7. The method according to claim 1-6, **characterised in that** microbiologically pure, preferably sterile, water is used to produce water and/or ice in the mixer (5).

8. The method according to claim 1-7, **characterised in that** in step (A) the waste material is removed by a conveyor belt (8).

9. The method according to claim 1-8, **characterised in that** the waste material, which is contaminants, manure and too small larvae, is then processed to produce a fertiliser, preferably as frass, is preferably transferred to a fertiliser production technological line and the waste material is processed to produce a fertiliser, preferably in the form of frass.

10. A liquefied feed material from *Hermetia illucens* insect biomass produced by the method as defined in claim 1-9.

11. The liquefied feed material from *Hermetia illucens* insect biomass according to claim 10, **characterised in that** it has the following parameters: dry matter is 15-30% w/w, it has a density in the range of 0.836-0.940 g/ml, has a viscosity at a temp. of 30°C in the range of 30-50 Pa*S, has a solid particle size diameter of up to 1 mm, total bacterial count is up to 10⁵ CFU/g.

12. Use of the liquefied feed material from *Hermetia illucens* insect biomass produced by the method as defined in claims 1-9 and/or the liquefied feed as defined in claims 10-11 for the production of food for companion animals, preferably dogs and cats.

13. The use according to claim 12, **characterised in that** the liquefied feed material from *Hermetia illucens* insect biomass is used in an amount of 10-50 wt% based on the total weight of the food.

14. The use according to claim 12-13, **characterised in that** the food is in a semi-liquid, solid, preferably dry form, preferably in the form of an animal treat.
